(19) Europäisches Patentamt

European Patent Office

Office européen des brevets

(11) **EP 1 630 557 A1**

(12) **EUROPÄISCHE PATENTANMELDUNG**

(43) Veröffentlichungstag:
01.03.2006 Patentblatt 2006/09

(51) Int Cl.:
*G01N 33/569* (2006.01)    *G01N 33/58* (2006.01)

(21) Anmeldenummer: 05008483.9

(22) Anmeldetag: **19.04.2005**

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **27.08.2004 DE 102004041659**

(71) Anmelder: **Institut
Virion/Serion GmbH
97076 Würzburg (DE)**

(72) Erfinder:
• **Hermann, Gerhard, Dr.
97076 Würzburg (DE)**

• **Kühlmann-Rabens, Ilona, Dr.
97993 Creglingen (DE)**
• **Leinfelder, Ulrich, Dr.
97076 Würzburg (DE)**
• **Reder, Sabine, Dr.
97941 Tauberbischofsheim (DE)**
• **Schobel, Uwe, Dr.
35576 Wetzlar (DE)**

(74) Vertreter: **Rudolph, Ulrike
Patentanwältin
In der Schanz 10
69198 Schriesheim (DE)**

(54) **Diagnostisches Testsystem für den Nachweis von Antikörpern gegen das Epstein Barr Virus (EBV)**

(57) Das diagnostische Testsystem für den Nachweis von Antikörpern gegen das Epstein Barr Virus umfaßt eine Kombination verschiedener Antigene als Sonden-Antigene. Diese Kombination enthält wenigstens ein EBV-spezifisches Antigen zum Nachweis spezifischer EBV-Antikörper und mindestens ein Antigen zum Nachweis heterophiler Antikörper. Die Antigene sind an Trägerpartikel gekoppelt, welche einzeln oder gruppenweise gleiche oder unterschiedliche Partikelgrößen aufweisen und/oder mit Fluoreszenzstoffen unterschiedlicher Emissionsspektren und/oder unterschiedlicher Intensitäten markiert sind, so daß mehrere Partikelpopulationen (jeweils bestehend aus einem oder mehreren gleichartigen Partikeln) vorliegen, von denen jede durch eine für sie spezifische Kodierung, nämlich die Kombination aus Größe, Fluoreszenzemissionsspektrum und Fluoreszenzinstensität charakterisiert ist. Jede Partikelpopulation ist mit einer anderen Art Antigen als Sonden- bzw. Detektor-Antigen beladen.

**Beschreibung**

Die Erfindung betrifft ein diagnostisches Testsystem für den Nachweis von Antikörpern gegen das Epstein Barr Virus (EBV), das eine Kombination verschiedener Antigene als Sonden-Antigene aufweist.

Das Epstein Barr Virus (EBV) ist der Erreger der infektiösen Mononukleose, daneben aber auch beteiligt an der Entstehung von mindestens drei Tumorerkrankungen des Menschen, nämlich dem Burkitt-Lymphom, dem Nasopharynxkarzinom und malignen Lymphomen bei immunsupprimierten Menschen, etwa Patienten nach Transplantationen oder HIV-Infizierten. Wie alle Herpesviren ist auch EBV in der Lage, in seinen Zielzellen, dem Epithel des Mund- und Rachenraums und den B-Lymphocyten, lebenslang nach einer Infektion zu persistieren. Im Epithel des Mund- und Rachenraums kommt es zunächst zu einer produktiven Infektion. Bei Ausreifen der infizierten Epithelzellen geht das Virus aus dem latenten in das replikative Stadium über und wird unter Cytolyse in den Rachen- und Mundraum abgegeben, so daß es lebenslang zur Ausscheidung infektiöser Viren kommen kann. Infizierte B-Lymphocyten hingegen werden "immortalisiert". Ohne Bildung neuer Viren werden die B-Zellen durch die persistierende Infektion zu lymphoblastoiden Zellen transformiert. Zusätzlich kommt es aufgrund einer polyklonalen Stimulation der B-Zellen zur Produktion von unspezifischen heterophilen Antikörpern und Autoantikörpern. Durch cytotoxische T-Zellen wird die Zahl der infizierten B-Zellen bis zur Einstellung eines dynamischen Gleichgewichts zwischen B-Zell-Proliferation und Lyse reduziert. Die Infektion wechselt nun in das latente Stadium.

Aufgrund der Abhängigkeit des Infektionsverlaufes von der individuellen Immunreaktivität des befallenen Organismus kann die EBV-Infektion ein breites Spektrum an klinischen Erscheinungen bieten. Störungen des Gleichgewichts zwischen T- und B-Zellen können darüber hinaus auch nach Ablauf einer EBV-Infektion EBV assoziierte Erkrankungen hervorrufen. In der Rheumatologie und Immunologie wird das Epstein-Barr-Virus als ein möglicher Auslöser von rheumatischen und immunologischen Erkrankungen (Autoimmunerkrankungen / Autoimmunreaktionen) diskutiert.

Da die Symptomatik der akuten EBV-Infektion mit vielen Krankheiten überlappen kann, die durch andere Erreger oder Ursachen hervorgerufen werden, besitzt die EBV-Diagnostik eine große differential-diagnostische Bedeutung. Ein früher Antikörper und damit Marker für eine primäre akute EBV-Infektion (Erstinfektion) ist das Anti-VCA-IgM. Es bleibt jedoch in der Regel nur wenige Wochen messbar. Erst etwa 3 - 6 Monate nach der akuten Infektion treten IgG-Antikörper gegen EBNA 1 auf. (Die EBNA 1-Exprimierung findet nur in der latenten Phase statt. EBNA 1 wird bei der Lyse der B-Lymphocyten durch die T-Zellen freigesetzt und führt dann zu einer reaktiven Antikörperbildung.) Der Nachweis von Anti-EBNA 1 schließt eine frische Infektion aus und kann einen Hinweis auf eine chronische Infektion geben. IgG-Antikörper gegen VCA treten verzögert auf und bleiben lebenslang erhalten (Persistenz) Beim Immungesunden ist nach Ablauf einer Infektion nur Anti-VCA-IgG und Anti-EBNA 1 nachweisbar, IgM-Titer und Anti-Early-Antigen sind in der Regel negativ. Das Anti-EBNA2 ist in der frühen Phase einer frischen Infektion nachweisbar. Ein abnehmendes Verhältnis des Anti-EBNA 1/Anti-EBNA 2-Quotienten wird als Zeichen für eine Reaktivierung gewertet.

Bei der EBV-Serologie besteht das Problem, daß sie ein hohes Maß an Variabilität aufweist. Durch Verwendung der Antikörper bzw. Marker VCA-IgG, VCA-IgM und Anti-EBNA-1 können nicht alle Fälle des Verdachts einer EBV-Infektion eindeutig geklärt werden. Beispielsweise zeigen nicht alle akuten EBV-Infektionen eine nachweisbare VCA-IgM-Antwort, so daß diagnostische Test, die allein auf der Bestimmung von VCA-IgG und VCA-IgM beruhen, zu einer falschen Schlußfolgerung führen können. Es kommt auch vor, daß eine VCA-IgM-Antwort monatelang persistiert und dadurch eine frische Infektion vortäuscht. Ein positiver Nachweis von Anti-EBNA-1 ist ein Indiz für eine abgelaufene EBV-Infektion und die Kombination mit einem positiven Nachweis von VCA-IgG ist praktisch ein Beweis dafür. Ein negativer Nachweis von Anti-EBNA-1 bei gleichzeitig positivem Nachweis von VCA-IgG schließt eine akute EBV-Infektion aus. Derselbe Befund kann auch durch sekundären Anti-EBNA-1-Verlust bei Immunsuppression verursacht sein. Außerdem entwickeln rund 5 % der Infizierten niemals nachweisbares Anti-EBNA-1 und täuschen damit lebenslang die serologische Situation einer akuten EBV-Infektion vor. Die Abgrenzung primärer Anti-EBNA-1-Negativität bei akuter EBV-Infektion einerseits, von Anti-EBNA-1-Verlust und fehlender Anti-EBNA-1-Bildung andererseits, stellt das größte Problem der EBV-Serologie dar. Insbesondere in der Frühphase einer EBV- Erkrankung ist die Gefahr gegeben, dass eine EBV-Infektion als solche (inapparentes Krankheitsbild) übersehen wird, da herkömmlichen Antikörpertests (ELISA, Westernblot) je nach Herstellerfirma und Testverfahren eine hohe Fehlerquote insbesondere hinsichtlich der Sensitivität aufweisen können. Die unverzügliche diagnostische und korrekte Aussage nach einer Infektion ist jedoch äußerst wichtig, um den Übergang in ein späteres, schwieriger zu therapierendes Stadium zu vermeiden.

Um eine EBV-Infektion einigermaßen zuverlässig zu diagnostizieren, werden derzeit verschiedene Tests auf der Basis von Anti-EBV Antikörpern durchgeführt. Hierzu gehören insbesondere

(a) der Indirekte Immunfluoreszenztest (IFT) und der Antikomplement-Immunfluoreszenztest (ACIF), mit dem die genannten EBV-Antikörper qualitativ, jedoch nicht quantitativ anhand von Fluoreszenzmarkierungen nachgewiesen werden können (ACIF vor allem zum Nachweis von Anti-EBNA 2 IgG);
(b) der ELISA-Test, mit dem die genannten Anti-EBV Antikörper qualitativ und quantitativ nachgewiesen werden können, wobei insbesondere zwischen den EBV-Antikörpern bei Vorliegen einer akuten EBV-Infektion und den Antikörpern bei Vorliegen einer chronischen, zurückliegenden oder reaktivierten EBV-Infektion unterschieden werden kann;

(c) der Immunblot / Westernblot / Lineblot, mit dem ebenfalls die genannten Anti-EBV Antikörper qualitativ und quantitativ nachgewiesen werden können, wobei insbesondere zwischen den EBV-Antikörpern bei Vorliegen einer akuten EBV-Infektion und den Antikörpern bei Vorliegen einer chronischen, zurückliegenden oder reaktivierten EBV-Infektion unterschieden werden kann;

(d) der Paul-Bunnell-Test, mit dem unspezifische heterophile Antikörper infolge einer EBV-Infektion anhand Agglutination nachgewiesen werden können;

Die Verfahren (a) bis (c) sind spezifische EBV-Antikörper-Nachweistests. Sie haben den Nachteil, daß jeweils in einem Arbeitsgang nur eine Art Antikörper bzw. nur Antikörper einer bestimmten Immunglobulinklasse analysiert werden kann. Solche diagnostischen Testverfahren sind deshalb hinsichtlich der diagnostischen Aussage stets eine limitierte Methode. In der Regel können Krankheitsbilder anhand einzelner Kriterien nicht voneinander abgegrenzt werden, und eine Aussage über den Immunstatus des betroffenen Patienten ist anhand solcher Einzeltests nicht möglich. Der Immunoblot bietet zwar den gleichzeitigen Nachweis verschiedener EBV-spezifischer Antikörper an, aber jeweils immer nur für eine bestimmte Immunglobulinklasse.

Der Paul-Bunnell-Test ist ein unspezifischer Schnelltest zum Nachweis der infektiösen Mononukleose und basiert auf dem Phänomen, daß bis zu 90% aller Patienten mit infektiöser Mononukleose im Serum einen hohen Titer von heterophilen Antikörpern aufweisen, welche mit Antigenen reagieren, die an ihrer Entstehung nicht beteiligt sind. Atypische und milde Krankheitsverläufe ergeben jedoch häufig negative Testbefunde, insbesondere bei Kindern. Der Paul-Bunnell-Test stellt daher ein nur gering empfindliches Nachweissystem für EBV-Infektionen dar und ist für humane Patienten unter 10 Jahren nicht geeignet.

Aus der DE 33 22 373 C2 und der EP 0 126 450 ist ein Testsystem zum simultanen Nachweis mehrerer verschiedener Antigene und/oder Antikörper aus einer Probe beschrieben, bei dem Trägerpartikel anstelle der bekannten ELISA-Kavitäten oder (Immuno-/Wester-/Line-)Blotzonen verwendet werden. Diese Trägerpartikel sind mit Detektor- bzw. Sonden-Antikörpern und/oder -Antigenen beschichtet. Die Partikel sind außerdem einzeln oder gruppenweise mit Fluoreszenzfarbstoffen unterschiedlicher Emissionsspektren und/oder mit unterschiedlichen Quantitäten wenigstens eines Fluoreszenzfarbstoffes und/oder durch unterschiedliche Partikelgrößen markiert, so daß letztendlich mehrere Partikelpopulationen (jede einzelne bestehend aus einem oder mehreren gleichartigen Partikeln) vorliegen, von denen jede durch eine für sie spezifische Kodierung, nämlich die Kombination aus Größe, Fluoreszenzfarbstoffart und Fluoreszenzfarbstoffmenge, charakterisiert ist. Jede Partikelpopulation (Partikelset) ist zudem mit einer anderen Art von Sonden-Antigenen beladen. Eine Mischung solcher Partikelpopulationen wird mit derjenigen Flüssigkeit (z.B. Patientenserum) gemischt, die die zu untersuchenden bzw. nachzuweisenden Antikörper (Proben-Antikörper) enthält. Anschließend werden die Reaktionsschritte eines konventionellen Immunfluoreszenzverfahrens durchgeführt: Nach einer definierten Reaktionszeit, in der die nachzuweisenden Antikörper von den an die Partikel gekoppelten Antigenen gebunden werden, erfolgt die Identifizierung der gebundenen Antikörper durch Zugabe einer Flüssigkeit mit fluoreszenzmarkierten Antikörpern, die mit den nachzuweisenden bzw. zu untersuchenden Antikörpern spezies-spezifisch reagieren. Zum Schluß wird mittels eines Meßgerätes, z.B. eines Durchflußzytometers, jedes Partikel hinsichtlich seiner Kodierung, nämlich Größe und Fluoreszenz-Emmisionsspektrum und FluoreszenzIntensität, vermessen. Anhand dieser Meßdaten kann auf das Vorhandensein der in Frage stehenden bzw. zu untersuchenden Antikörper rückgeschlossen werden.

Der vorliegenden Erfindung liegt die Aufgabe zugrunde, ein Testsystem für die Diagnose von EBV-Infektionen bereitzustellen, das schnell durchzuführen ist, zuverlässige spezifische Ergebnisse liefert, und das den Nachweis und die Identifizierung aller relevanten Anti-EBV-Antikörper sowie die Bestimmung des EBV-Infektionsstatuses (Erstinfektion, Persistenz, Reaktivierung) in ein und demselben Arbeitsgang ermöglicht Eine Lösung dieser Aufgabe besteht in der Bereitstellung eines Testsystems der eingangs genannten Art, das dadurch gekennzeichnet ist, daß die Kombination (i) wenigstens ein EBV-spezifisches Antigen zum Nachweis spezifischer EBV-Antikörper und mindestens ein Antigen zum Nachweis heterophiler Antikörper (beispielsweise ein sogenanntes Paul-Bunnell-Antigen) enthält, und daß (ii) die Antigene an Trägerpartikel (vorzugsweise adsorptiv) gekoppelt sind, wobei (iii) diese Trägerpartikel einzeln oder gruppenweise gleiche oder unterschiedliche Partikelgrößen aufweisen und/oder mit Fluoreszenzstoffen unterschiedlicher Emissionsspektren und/oder unterschiedlicher Intensitäten markiert sind, so daß mehrere Partikelpopulationen (jeweils bestehend aus einem oder mehreren gleichartigen Partikeln) vorliegen, von denen jede durch eine für sie spezifische Kodierung, nämlich die Kombination aus Größe, Fluoreszenzemissionsspektrum und Fluoreszenzintensität charakterisiert ist, und wobei (iv) jede Partikelpopulation mit einer anderen Art Antigen als Sonde- bzw. Detektor-Antigen beladen ist.

Dieses Testsystem bietet den Vorteil, daß auf die Fragen nach dem Vorliegen einer EBV Virusinfektion und gegebenenfalls nach dem Verlauf und dem Status der Infektion (akut und Erstinfektion, akut und reaktivierte Infektion, ruhende persistierende Infektion) eine aussagekräftige, serologische Antwort anhand eines einzigen Testlauf mit einer einzigen Serumprobe des betreffenden Patienten erhalten werden kann. Die gleichzeitige Analyse mehrerer Parameter - insbesondere Art und Menge von bestimmten spezifischen Antikörpern und von unspezifischen Antikörpern (z.B.

Paul-Bunnell-Antikörper und/oder sonstigen Elementen/Faktoren des Immunsystems (z.B. Komplement C3) - ermöglicht eine (insbesondere im Vergleich zu den herkömmlichen separaten Untersuchungsgängen) schnelle und kosteneffiziente Diagnose der verschiedenen EBV-Infektionsstadien, vor allem den Ausschluß einer Primärinfektion, und sie erlaubt gleichzeitig eine zuverlässige Bestimmung des EBV-Immunstatus, was insbesondere bei immunsuppressiven und immundefizienten Patienten oft von besonderer Wichtigkeit ist. Die simultane Messung von Art und Menge verschiedener Antikörper aus unter Umständen verschiedenen Immunglobulinklassen im Probenserum (Patientenserum) ermöglicht die Berechnung von Quotienten und anderen mathematischen und statistischen Größen (insbesondere auch eine Quantifizierung) und erlaubt damit weitere zuverlässige Aussagen über die Art des Infektionsstatus (siehe Tabelle 1 und Tabelle 3), insbesondere darüber, ob eine akute (Erstinfektion) oder abgelaufene (Persistenz) oder reaktive (Reaktivierung) oder chronische Krankheitsform vorliegt. Etwaige Antikörperpersistenzen, die in separaten Tests eine Unterscheidung von frischer oder zurückliegender Infektion erschweren, werden mit dem erfindungsgemäßen Testsystem sicher identifiziert, ohne die Aussagekraft des Testergebnisses negativ zu beeinflussen.

Das erfindungsgemäße Testsystem bietet so auch die Vorteile, dass eine differentialdiagnostische Abgrenzung zu anderen Herpesviren-Infektionen und Hepatitisähnlichen Erkrankungen (z.B. HSV, CMV, Rubella, Mumps, Hepatitis) vorgenommen werden kann, und daß EBV-assoziierte Leitsymptome und Erkrankungen frühzeitig erkannt werden können (z.B. Arthritis, Tonsillitis, Nasopharyngitis-Karzinom, Hepatitis, Meningitis, Laryngitis, Mononukleose, Lymphome, Pharyngitis, Kopfschmerzen, Fieber, Enantem, Lymphknotenschwellung, Tonsillitis, Enzephalitis, Arzneimittelexanthem). Der Nachweis einer akuten EBV-Infektion kann ausschlagendes Kriterium zum erkennen einer polyklonal stimulierten IgM-Antwort sein, welche durch die vorübergehende und zufällige Interaktion von EBV mit B-Zellen ausgelöst wird. EBV-Infektionen führen stets zu einer polyklonalen Stimulierung und könne mitunter serologisch andere Infektionen vortäuschen.

Aufgrund des Merkmals, daß die Antigen-Kombination zusätzlich nämlich ein Antigen zum Nachweis heterophiler Antikörper. d.h. einen Marker für die polyklonale Stimulation von B-Zellen umfaßt, insbesondere ein Glykoprotein der Erythrozytenmembran von Pferde- oder Schaf(Hammel) oder Rinder-Erythrozyten, welches sensitiv mit (auf) heterophile(n) Antikörper(n) reagiert, bietet das erfindungsgemäße Testsystem den Vorteil, daß in ein und demselben Testlauf gleichzeitig das Vorhandensein bestimmter definierter spezifischer EBV-Antikörper und das Vorhandensein unspezifischer heterophiler Antikörper als Indikator für eine akute infektiöse Mononukleosis überprüft bzw. nachgewiesen werden können.

Mit dem gleichzeitigen Nachweis des Vorliegens unspezifischer heterophiler Antikörper nach einer EBV Infektion anhand einer erhöhten Stimulation der B-Zellen kann eine direkte Aussage über eine akute oder länger zurückliegende chronische oder reaktive Infektion getroffen werden und damit auch eine Aussage, ob bei den Nachweisreaktionen der distinkten EBV-Proteine ein echt-positives Ergebnis vorliegt oder nicht. Eine solche Aussage ist allein aufgrund des Nachweises bestimmter Immunglobulin- bzw. Proteintypen nicht oder nur sehr eingeschränkt möglich, und eine komplette serologische Information erforderte daher bisher einen erheblich höheren Zeit- und Kostenaufwand.

Die alleinige Durchführung eines Paul-Bunnell-Tests erlaubt keine sichere Aussage zum Vorliegen oder Ausschluß einer frischen EBV-Infektion, da nur ca. 90% der frisch Infizierten im Paul-Bunnell-Test positiv reagieren, bei Kindern unter 10 Jahren das Testergebnis noch unzuverlässiger ist, und je nach Testsystem ein positives Ergebnis bis zu einem Jahr nach der Erstinfektion erhalten werden kann. Für eine sensitive und spezifische Aussage zur Diagnose einer EBV-Erkrankung ist deshalb eine Kopplung an eine EBV-Serologie zwingend notwendig.

In einer bevorzugten Ausführungsform ist wenigstens ein EBV-spezifisches Antigen als Virus-Capsid-Antigen (VCA) des Epstein Barr Virus realisiert, denn damit ist der Nachweis von aktiven, akuten EBV-Infektionen möglich (vgl. Tabelle 1 und Tabelle 3).

Vorzugsweise ist des weiteren wenigstens ein EBV-spezifisches Antigen als nukleäres Antigen (EBNA) des Epstein Barr Virus realisiert, denn damit ist der Nachweis von latent persistierenden Infektionen möglich (vgl. Tabelle 1 und Tabelle 3).

Weiter vorzugsweise umfaßt die erfindungsgemäße Sonden-Antigen-Kombination zusätzlich wenigstens ein Early Antigen (EA) des Epstein Barr Virus. Damit enthält das Testsystem alle bekannten und bedeutsamen Antigene des Epstein Barr Virus. Anhand der konkret nachgewiesenen EBV-Antikörper-Kombination in einer Patienten-Serumprobe und den in Tabelle 1, Tabelle 2 und Tabelle 3 aufgelisteten, bekannten Zusammenhängen zwischen EBV-Antigentyp, Antikörperart und Infektionsstatus kann direkt auf den Infektionsstatus des betreffenden Patienten geschlossen werden.

Zum Nachweis heterophiler Antikörper wird ein Glykoprotein der Erythrozytenmembran von Pferde- oder Schaf (Hammel) oder Rinder-Erythrozyten gemäß dem im Stand der Technik bekannten Paul-Bunnell-Test vorgeschlagen.

Aufgrund der in den Tabellen 1, 2 und 3 dargestellten Erkenntnisse im Stand der Technik wird vorgeschlagen, daß als Virus Capsid Antigen (VCA) wenigstens das VCA-p18 Protein zum Einsatz kommt und weiter vorzugsweise außerdem das VCA-p23-Protein.

Als nukleäres Antigen (EBNA) sollte das EBNA 1 (p72-) Protein oder das EBNA 2 (p55-) Protein eingesetzt werden, vorzugsweise beide gleichzeitig, weil damit das Mengenverhältnis zwischen diesen beiden EBNA-Proteinen bestimmt werden kann, und ein Absinken des Quotienten Anti-EBNA1 / Anti-EBNA2 auf kleiner 1 ein eindeutiges Indiz für eine Reaktivierung einer EBV-Infektion ist (vgl. Tabellen 1 und 2).

Als Early Antigene (EA) sollte das EA-p54-Protein und/ oder das EA-p138-Protein eingesetzt werden. Hohe Anti-EA-Titer (IgA-Antikörper) sind ein Indikator für das Vorliegen spezieller Karzinomerkrankungen, insbesondere für das NasopharynxKarzinom in Asien.

In einer ebenfalls bevorzugten Ausführungsform umfaßt das Testsystem zusätzlich zu den EBV-Antigenen mindestens ein Antigen zum Nachweis von EBNA-spezifischen, - vorzugsweise EBNA1- oder EBNA2- spezifischen -, komplementbindenden Antikörpern. Der Antikomplement-Nachweis dient unter anderem zur Differenzierung zwischen einer primär, akuten (0-4 Wochen), einer primär, kürzlich erfolgten (4-12 Wochen) und/oder einer früher erfolgten (>12 Wochen) Infektion bzw. einer Reaktivierung. Infolgedessen hat diese Ausführungsform des Testsystems den Vorteil, daß eine primäre Infektion, eine Reaktivierung oder eine früher erfolgte Infektion besser differenziert und quantifiziert werden können.

Als Material für die Trägerpartikel hat sich in der Praxis Latex als besonders geeignet erwiesen. Eine bevorzugte Ausführungsform des erfindungsgemäßen Testsystems zeichnet sich folglich dadurch aus, daß die Trägerpartikel in Form von Latexpartikel realisiert sind.

In der praktischen Erprobung hat sich außerdem eine Variante als sehr geeignet erwiesen, bei der mehrere Partikelpopulationen vorliegen, wobei die Partikel aller Populationen die gleiche Partikelgröße, vorzugsweise zwischen 1 $\mu$m und 10 $\mu$m, besitzen und mit demselben Fluoreszenzfarbstoff markiert sind, aber jede Partikelpopulation eine andere Fluoreszenzintensität aufweist.

In einer weiteren, für die praktische Anwendung besonders bevorzugten Ausführungsform umfasst das erfindungsgemäße diagnostische Testsystem zusätzlich (erstens) für jeden nachzuweisenden (d.h. zu den Sondenantigenen komplementären) Antikörper die Angabe einer Referenzkurve, die die Korrelation (das Verhältnis) zwischen Antikörperkonzentration im Probenvolumen und gemessener Fluoreszenzsignalstärke wiedergibt, und (zweitens) ein Standardserum, welches alle nachzuweisenden, d.h. zu den Sondenantigenen komplementären Antikörper in bekannten Konzentrationen enthält. Die Antikörper-spezifischen Referenzkurven sind durch die Formel

$$\text{Count} = A + \frac{D - A}{1 + e^{B(C - \ln \text{Konz.})}}$$

bzw.:

$$\text{Konz.} = \exp\{C - \ln[(D-A)/(\text{Count} - A) - 1]/B\}$$

mit:

Count = Median der gemessenen Fluoreszenzsignale
A = untere Asymptote der Referenzkurve
B = Steigung der Referenzkurve
C = Wendepunkt der Referenzkurve
D = obere Asymptote der Referenzkurve

beschriebenen. Diese Referenzkurvenformel ist im Stand der Technik bekannt, beispielsweise aus DE 36 39 279 C3 in Kombination mit Van Loon und Van der Veen in J. Clin. Pathol. 33, 635-639, 1980 und Van Loon et al., J. Clin. Pathol. 34, 1981, 665-669 und aus Dundley, R.A., Edwards, P., Ekins, R.P., Finney, D.J., McKenzie, J.G.M., Raab, G.M., Rodbard, D., and Rodgers, R.P.C. (1985) "Guideline for immunoassay data processing." Clin. Chem. 31, 1264-1271.

Die Referenzkurven-spezifischen Parameter A, B, C, und D werden experimentell mit der dem Fachmann bekannten und geläufigen Methode der Titration (geometrische Verdünnungsreihe) von Kalibratorseren (z.B. internationalen oder nationalen Standards) ermittelt. Vorzugsweise wird für jeden nachzuweisenden Antikörper (Analyten) und für jede seiner Herstellungschargen die Referenzkurve in mehrfachen Testläufen unter Einhaltung optimaler Bedingungen erstellt. Damit geht der Vorteil einher, dass die kosten- und zeitintensive Erstellung solcher Referenzkurven auf Seitens des Anwenders, insbesondere des Arztes oder des Laborpersonals, entfallen kann.

Mit Hilfe des Standardserums werden die Antikörper-spezifischen Referenzkurven rekalibriert, d.h. an die tatsächlichen, aktuellen Testbedingungen angepaßt.

Bei der Rekalibrierung (Tageskorrektur) wird das Verhältnis zwischen Ist-Zustand (IstWert) und Soll-Zustand (Sollwert) ermittelt, das wie folgt in die o.g. Referenzkurvenformel einfließt:

$$\text{Konz.} = \exp\{C\text{-}\ln[(D\text{-}A)/((\text{Count-KLW})*STD_{\text{soll}}/(STD_{\text{ist}}\text{-KLW})\text{-}A)\text{-}1]/B\}$$

mit:

KLW = Konjugatleerwert
$STD_{\text{soll}}$ = Sollwert des Standards
$STD_{\text{ist}}$ = aktuelle Tageswert des Standards

In der praktischen Anwendung erfolgt die jeweilige Ausrechnung dieser Formel vorzugsweise mit Hilfe von Rechnern und entsprechender Auswertesoftware, so dass die errechneten Ergebnisse der Quantifizierung nahezu zeitgleich mit den tatsächlich ermittelten Messdaten des durchgeführten Diagnosetestlaufs vorliegen.

Mit einer solchen Kalibrierung werden Testschwankungen ausgeglichen und die Qualität des Testlaufes überprüft.

Zusammengefaßt bietet das erfindungsgemäße diagnostische Testsystem vor allem die Vorteile einer raschen und kostengünstigen Routine-Serologie zum Erkennen des EBV-Status durch den gleichzeitigen Nachweis von (a) spezifischen Anti-EBV-Antikörpern aller Immunklassen und (b) unspezifischen heterophilen Antikörpern. Der Nachweis von spezifischen Anti-EBV-Antikörpern ist insbesondere auf die Anti-VCA als Marker für frische Infektion (Seropositivität) und/oder Anti-Early-Antigen (EAD und/oder EA-R) als Marker für die aktive Phase (Virusproduktion) und/oder Anti-EBNA1- und Anti-EBNA2 zum Ausschluss einer frischen Infektion und Erkennung einer Reaktivierung gerichtet. Vorzugsweise umfaßt das Testsystem außerdem die Angabe einer Referenzkurve, die die Korrelation (das Verhältnis) zwischen Antikörperkonzentration im Probenvolumen und gemessener Fluoreszenzsignalstärke wiedergibt, und ein Standardserum, welches jeden der nachzuweisenden Antikörper in bekannten Konzentration enthält, und welches zur Rekalibrierung aller vorgegebenen Referenzkurven geeignet und vorgesehen ist.

Die Erfindung wird im folgenden anhand von Ausführungsbeispielen näher erläutert.

**Beispiel 1: Partikelkodierung**

Im Unterscheid zur ELISA-Technik, bei der die Kavitätswände der Mikrotiterplatte zur adsorptiven Kopplung der Analyte bzw. Antikörper dienen, werden in dem erfindungsgemäßen Testsystem Trägerpartikel, vorzugsweise Latexpartikel, mit einem Durchmesser von beispielsweise 4,0 µm als Festphase eingesetzt. Die prinzipiell ununterscheidbaren Partikel sind hier im Beispiel mit verschiedenen, distinkten Intensitäten desselben roten Fluorophors gefärbt, wodurch ein Sortiment von sieben unterschiedlich stark rot gefärbten Latexpartikel-Sets (Partikelpopulationen) erhalten wird.

Jedes Set (jede Partikelpopulation) wird mit unterschiedlichen Antigenen beschichtet, das heißt, an jedem Partikelset wird eine bestimmte Art Antigen kovalent oder nicht-kovalent immobilisiert, beispielsweise wie folgt:

Partikelset/Partikelpopulation mit Rot-Intensität 1 : mit EBV-VCA-p18
Partikelset/Partikelpopulation mit Rot-Intensität 2 : mit EBV-VCA-p23
Partikelset/Partikelpopulation mit Rot-Intensität 3 : mit EBV-EBNA 1-p72
Partikelset/Partikelpopulation mit Rot-Intensität 4: mit EBV-EBNA 2-p55
Partikelset/Partikelpopulation mit Rot-Intensität 5 : mit EBV-EA-p54
Partikelset/Partikelpopulation mit Rot-Intensität 6: mit EBV-EA-p138
Partikelset/Partikelpopulation mit Rot-Intensität 7: mit Rinder-Erythrozyten (Paul-Bunnell-Antigen)

Um nun mehrere Untersuchungen gleichzeitig in einem Testansatz durchführen zu können, werden die antigenbeschichteten Partikel-Sets (Partikelpopulationen) zu einem sogenannten Test-Cocktail gemischt. Ein solcher Test-Cocktail (d.h. eine solche konkrete Partikel-Set-Mischung), der (die) beispielsweise die Antigene EBV-VCA-p18, EBV-VCA-p23, EBV-EBNA 1-p72, EBV-EBNA 2-p55, EBV-EA-p54, EBV-EA-p138, und Rinder-Erythrozyten (Paul-Bunnell-Antigen) oder Fragmente dieser Antigene umfaßt, oder auch nur einige dieser Antigene und/oder andere EBV-Antigene aufweist, stellt eine Ausführungsvariante des erfindungsgemäßen Testsystems dar.

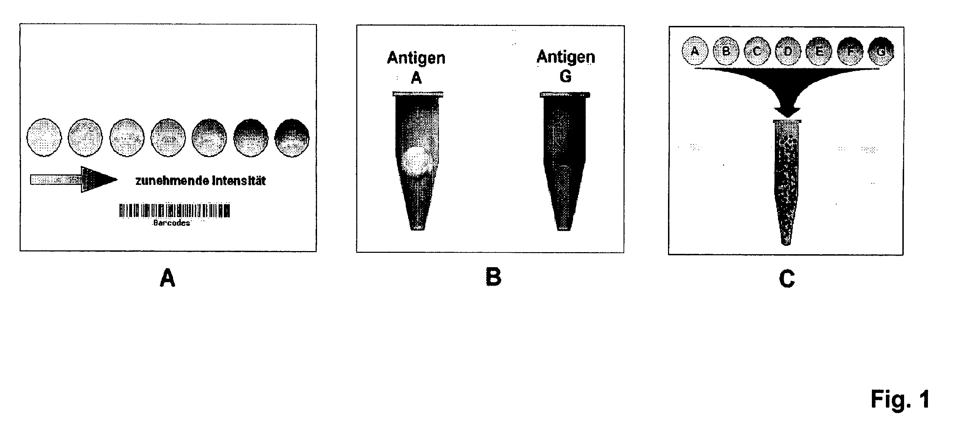

In Fig. 1 sind die einzelnen Schritte der Partikelkodierung und Testsystemherstellung noch einmal bildlich dargestellt. Fig. 1 (A) zeigt die Kodierung der Partikel mit dem roten Fluorophor in verschiedenen Intensitätsstufen. Fig. 1 (B) zeigt die Beschichtung der Partikel mit dem Antigen. Fig. 1 (C) zeigt die Mischung der beschichteten Partikel zu dem Test-Cocktail.

### Beispiel 2: Durchführung eines EBV-Tests

Eine vorzugsweise gemäß Beispiel 1 hergestellte Partikel-Set-Mischung wird mit einer bestimmten Menge Patientenserum gemischt und inkubiert. Während der Inkubation binden vorhandene Antikörper aus dem Patientenserum ("Patientenantikörper") an diejenigen Latexpartikel, die mit dem ihrer Spezifität entsprechenden Antigen beschichtet sind. Anschließend wird der Probe (-nlösung) ein Konjugatantikörper zugegeben, beispielsweise ein gegen humane Antikörper gerichteter, polyklonaler Ziegen-Antikörper, der mit dem an die Latexpartikel gebundenen Patientenantikörper reagiert, das heißt an diesen bindet. Dieser Konjugatantikörper ist vorzugsweise mit einem weiteren Fluoreszenzfarbstoff markiert, wobei dieser weitere Fluoreszenzfarbstoffe ein anderes Emissionsspektrum oder zumindest eine andere Farbstoffintensität aufweist als der Fluoreszenzfarbstoff, mit dem die Partikel(sets) markiert sind. Hier im Beispiel wurde ein grüner Fluorophor gewählt.

Die Menge gebundener Konjugatmoleküle ist proportional zur Antikörperkonzentration.

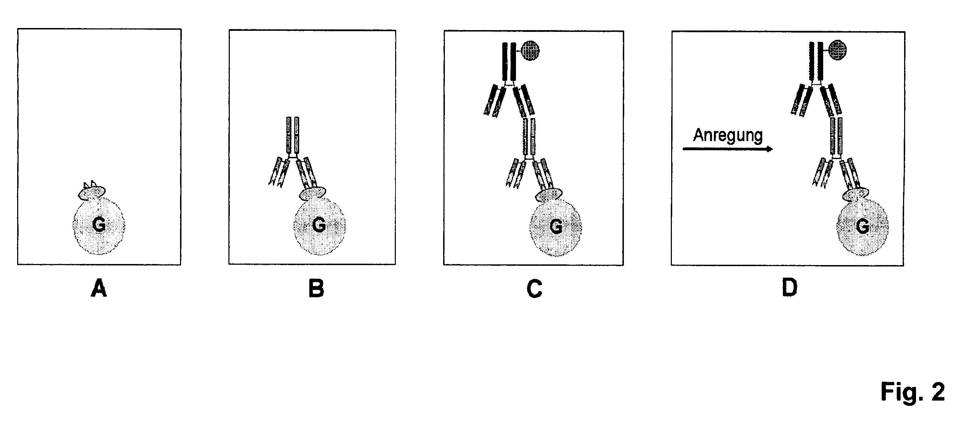

In Fig. 2 sind die einzelnen Schritte der Testdurchführung noch einmal bildlich dargestellt. Fig. 2 (A) zeigt die Vorlage des EBV-Test-Cocktails. Fig. 2 (B) zeigt die Zugabe des Patientenserums mit den Patienten-spezifischen Patientenantikörpern. Fig. 2 (C) zeigt die Zugabe des Konjugatantikörper (mit grüner Fluoreszenz). Fig. 2 (D) zeigt das Reaktionsgefäß während der Detektion im Durchflußzytometer.

### Beispiel 3: Testauswertung

Das vorzugsweise gemäß Beispiel 1 und Beispiel 2 hergestellte und eingesetzte Testsystem trägt nun je Latexpartikel-Set eine variable Menge der Konjugat-Fluoreszenz auf seiner Oberfläche und wird vorzugsweise mit Hilfe eines für Partikel-Messungen spezialisierten Durchflußzytometers, eines sogenannten Partikel-Fluoreszenz-Durchfluß-Meßgeräts, analysiert.

Diese Meßgeräte analysieren individuelle Latexpartikel nach ihrer Fluoreszenz und können gleichzeitig drei verschiedene Fluoreszenzfarben (Orange, Rot, Dunkelrot) unterscheiden.

Hier im Beispiel analysiert das Durchflußzytometer zum einen anhand der Rot-Emission die verschiedenen Partikelsets und damit die verschiedenen Antigene und zum anderen anhand der Grün-Emission die Menge gebundener Konjugat-Antikörper und damit die Menge an gebundenem Patientenantikörper für jedes Partikelset d.h. für jedes spezifische Antigen.

Ein großer Vorteil dieser Durchflußzytometrie-Analytik ist darin zu sehen, daß entsprechende Geräte meist bereits bei den Anwendern vorhanden sind. Darüber hinaus wird die diagnostische Leistungsfähigkeit der immunologischen Teste durch die erhöhte Sensitivität der Fluoreszenz-Signalgebung im Vergleich zur ELISA-Farbreaktion erhöht. Mit Hilfe einer Software klassifiziert das Gerät die Fluoreszenz jedes Partikels zu den passenden Partikel-Sets und ordnet die entsprechenden Analytbestimmungen zu. Die mittlere Konjugat-Fluoreszenz je Partikel-Set ist ein Maß für die Konzentration des entsprechenden Analyten in der Serumprobe.

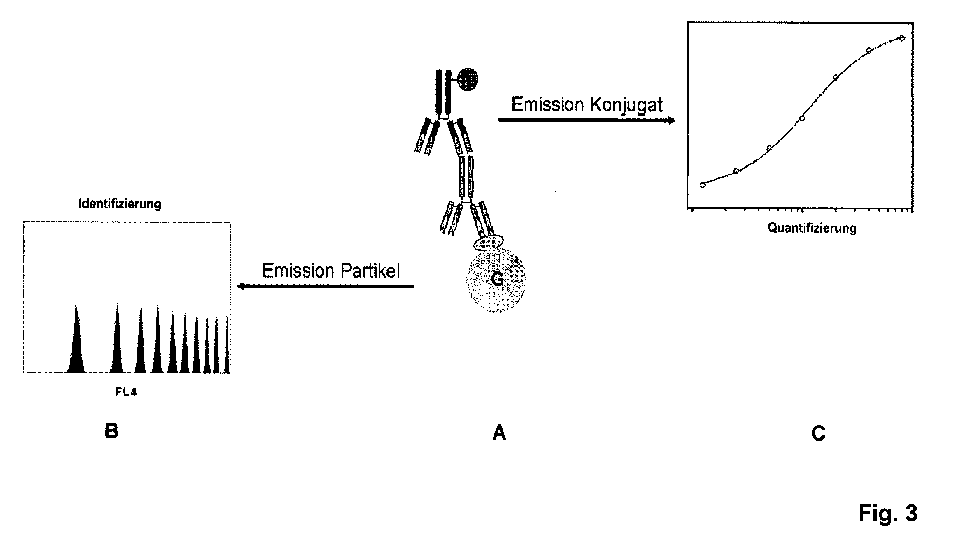

In Fig. 3 sind die einzelnen Schritte der Testauswertung noch einmal bildlich dargestellt. Fig. 3 (A) zeigt das Reaktionsgefäß während der Detektion im Durchflußzytometer. Fig. 3 (B) zeigt das Detektionsbild der roten Emission, und erlaubt damit die Identifikation der verschiedenen, mit spezifischen Antigenen beladenen Partikelpopulationen. Fig. 3 (C) zeigt das Detektionsbild der grünen Emission und erlaubt damit die Identifizierung und Zuordnung der Menge an Konjugatantikörper je Partikelpopulation.

**Tabelle 1: Zusammenhänge zwischen EBV Antigen-Typ, Antikörperart und Infektionsstatus:**

| Antigen | Antikörper | Infektionsstatus |
|---|---|---|
| Virus-Capsid-Antigen VCA | | aktive, akute Infektion |
| | AntiVCA-IgM | frische Infektion (selten bei reaktivierter Infektion) - meist zeitgleich mit VCA-IgG, häufig verzögert, selten früher, im Einzelfall fehlend! - kann bis zu 1 Jahr persistieren, selten länger - kann bei Reaktivierungen wieder auftreten |

Tabelle fortgesetzt

| Antigen | Antikörper | Infektionsstatus |
|---|---|---|
| | Anti-VCA-IgG | frische und persistierende Infektion (Seropositivität) - meist schon in Inkubationsphase nachweisbar |
| | Anti-VCA-IgA | frische und persistierende Infektion (gelegentlich bei reaktivierter Infektion) - hochtitrig bei Nasopharynxcarzinom NPC - auch bei lymphoproliferativen Erkrankungen nach Transplantationen nachweisbar |
| Early Antigen EA | | aktive, akute Infektion - Immunfluoreszenzserologische Unterscheidung in EA-D (diffus) und EA-R (restricted) |
| | Anti-EA-D-IgG | aktive, akute Infektion - Anstieg in akuter Phase (70 - 80 %) - i.d.R. nach 3-6 Mon. nicht mehr nachweisbar - Wiederauftreten bei Reaktivierung |
| | Anti-EA-R-IgG | frische Infektion - Anstieg meist erst in Rekonvaleszenz (Ausnahme: Kleinkinder) - Nachweis der akuten Infektion bei Kleinkindern, wenn Paul-Bunnell-Test und Anti-EA-D-Nachweis negativ - hochtitrig beim Burkitt-Lymphom |
| | Anti-EA-IgA | aktive, akute chronisch reaktivierte Infektion - positiv beim Nasopharynxcarzinom |
| Nuclear Antigen 1-6 EBNA1-6 | | latente persistierende Infektion |
| | Anti-EBNA 1-IgG | latente persistierende Infektion - Nachweis schließt Erstinfektion i.d.R. aus - nach abgelaufener Infektion meistens (Ausn: Immunsuppression!) lebenslang nachweisbar |
| | Anti-EBNA 2-IgG | latente persistierende Infektion - z. T. schon bei frischer Infektion nachweisbar - Absinken des Quotienten Anti-EBNA1 / Anti-EBNA2 auf <1 zeigt Reaktivierung an |

**Tabelle 2: Zusammenhänge zwischen Infektionsstatus und EBV-Antikörper-Kombination:**

| Infektionsstatus | Antikörper |
|---|---|
| frische Infektion | Anti-VCA-IgM, v.a. Anti-EBV-p18<br>Anti-EA-IgG, v.a.Anti-EBV-p54 und Anti-EBV-p138 |
| zurückliegende Infektion (nicht aktiv, latent, kein Krankheitsbild, inapparent) | (Anti-VCA-IgM, v.a. Anti-EBV-p18) < 6 Monate<br><br>Anti-VCA-IgG, v.a. Anti-EBV-p23<br>Anti-EBNA1-IgG (Anti-EBV-p72-IgG) |
| reaktivierte Infektion | Anti-VCA-IgM, v. a. Anti-EBV-p18<br>Anti-VCA-IgG, v.a. Anti-EBV-p23<br>Anti-EA-IgG, v.a.Anti-EBV-p54 und Anti-EBV-p138<br>Anti-EBNA2-IgG (Anti-EBV-p55-IgG) |
| abgelaufene Infektion | Anti-EBNA1-IgG (Anti-EBV-p72-IgG) |

**Tabelle 3: Typische Reaktionsmuster:**

| Reaktivität | Befund |
|---|---|
| Kein spezifische Befund in allen Immunglobulinklassen | EBV-negativ |
| IgG: EBNA1(p72) und VCA(p18) IgG negativ (VCA(p23) und EA(p54/p138) können positiv sein) IgM: EA (p54 und/ oder p138) positiv; VCA (p18/p23) möglich | Erstinfektion (**frische Infektion**) Infektiöse Mononukleose |
| IgG: EBNA1(p72) IgG und/oder VCA(p18) positiv, VCA (p23) häufig positiv; EA Titer schwach positiv möglich IgM: negativ | Abgelaufene, nicht mehr akute Infektion **(zurückliegende Infektion)** |
| IgG: wie bei einer abgelaufenen Infektion, EA(p54/p138) aber schwach vorhanden IgM: EA und/oder VCA schwach positiv | Sekundäre Reaktivierung (klinisch inapparent, kein Krankheitsbild) |
| IgG: hohe Titer gegen alle Antigene, in Ausnahmen anti-EBNA1(p72) Verlust IgM: EA und VCA Titer können schwach positiv sein IgA: deutliche Reaktivitäten mit EA und VCA | **Reaktivierung:** d. h. es liegt eine Wiederaufnahme der lytischen Vermehrung in einer latent infizierten Person vor. Das Ausmaß kann von kurzer Dauer sein (kurzzeitige Störung des Immunsystems, z. B. durch Immunsuppressiva) |
| Serologisches Bild wie bei Reaktivierung (hohe IgA Titer) | Nasopharynxkarzinom und EBV-relevante Lymphome |

**Patentansprüche**

1. Diagnostisches Testsystem für den Nachweis von Antikörpern gegen das Epstein Barr Virus, das eine Kombination verschiedener Antigene als Sonden-Antigene aufweist, **dadurch gekennzeichnet,**

   - **dass** die Kombination wenigstens ein EBV-spezifisches Antigen zum Nachweis spezifischer EBV-Antikörper und mindestens ein Antigen zum Nachweis heterophiler Antikörper enthält,
   - und **dass** die Antigene an Trägerpartikel gekoppelt sind,

     -- wobei diese Trägerpartikel einzeln oder gruppenweise gleiche oder unterschiedliche Partikelgrößen aufweisen und/oder mit Fluoreszenzstoffen unterschiedlicher Emissionsspektren und/oder unterschiedlicher Intensitäten markiert sind, so daß mehrere Partikelpopulationen (jeweils bestehend aus einem oder mehreren gleichartigen Partikeln) vorliegen, von denen jede durch eine für sie spezifische Kodierung, nämlich die Kombination aus Größe, Fluoreszenzemissionsspektrum und Fluoreszenzinstensität charakterisiert ist,
     -- und wobei jede Partikelpopulation mit einer anderen Art Antigen als Sonden- bzw. Detektor-Antigen beladen ist.

2. Testsystem nach Anspruch 1, **dadurch gekennzeichnet,**
   **dass** wenigstens ein EBV-spezifischesAntigen als Virus-Capsid-Antigen (VCA) des Epstein Barr Virus realisiert ist.

3. Testsystem nach Anspruch 1 oder 2, **dadurch gekennzeichnet,**
   **dass** wenigstens ein EBV-spezifisches Antigen als nukleäres Antigen (EBNA) des Epstein Barr Virus realisiert ist.

4. Testsystem nach einem der Ansprüche 1 bis 3, **dadurch gekennzeichnet,**
   **dass** wenigstens ein EBV-spezifisches Antigen als Early Antigen (EA) des Epstein Barr Virus realisiert ist.

5. Testsystem nach einem der Ansprüche 1 bis 4, **dadurch gekennzeichnet,**
   **daß** das Antigen zum Nachweis heterophiler Antikörper ein Glykoprotein der Erythrozytenmembran von Pferde- oder Schaf(Hammel) oder Rinder-Erythrozyten ist.

6. Testsystem nach einem der Ansprüche 2 bis 5, **dadurch gekennzeichnet, daß** es sich bei dem Virus Capsid Antigen (VCA) um das VCA-p18 Protein handelt.

7. Testsystem nach einem der Ansprüche 2 bis 5, **dadurch gekennzeichnet, daß** es sich bei dem Virus Capsid Antigen (VCA) um das VCA-p18 Protein und um das VCA-p23 Protein handelt.

8. Testsystem nach einem der Ansprüche 3 bis 7, **dadurch gekennzeichnet, daß** es sich bei dem nukleären Antigen (EBNA) um das EBNA1 (p72-) Protein handelt.

9. Testsystem nach einem der Ansprüche 3 bis 7, **dadurch gekennzeichnet, daß** es sich bei dem nukleären Antigen (EBNA) um das EBNA2 (p55-) Protein handelt.

10. Testsystem nach einem der Ansprüche 4 bis 9, **dadurch gekennzeichnet, daß** es sich bei dem Early Antigen (EA) um das EA-p54-Protein und/oder um das EA-p138-Protein handelt.

11. Testsystem nach einem der Ansprüche 1 bis 10, **dadurch gekennzeichnet, dass** mindestens ein Antigen zum Nachweis von EBNA2-spezifischem komplementbindenden Antikörpern vorgesehen ist.

12. Testsystem nach einem der Ansprüche 1 bis 9, **dadurch gekennzeichnet, daß** die Trägerpartikel Latexpartikel sind.

13. Testsystem nach einem der Ansprüche 1 bis 10, **dadurch gekennzeichnet, daß** mehrere Partikelpopulationen vorliegen, wobei alle Partikel die gleiche Größe aufweisen, alle Partikel mit demselben Fluoreszenzfarbstoff markiert sind, aber jede der Partikelpopulationen eine andere Fluoreszenzintensität aufweist.

14. Testsystem nach einem der Ansprüche 1 bis 11, **dadurch gekennzeichnet, daß** die Trägerpartikel eine Größe von 1 $\mu$m bis 10 $\mu$m aufweisen.

15. Testsystem nach einem der Ansprüche 1 bis 14, **dadurch gekennzeichnet, daß** es zusätzlich umfaßt:

> (i) für jeden der zu den Sondenantigenen komplementären Antikörper die Angabe einer Referenzkurve, welche das Verhältnis zwischen Antikörperkonzentration im Probenvolumen und gemessener Fluoreszenzsignalstärke wiedergibt und durch die Funktion:

$$Count = A + \frac{D - A}{1 + e^{B(C - \ln Konz.)}}$$

> bzw.:

$$Konz. = \exp\{C - \ln[(D-A)/(Count - A) - 1]/B\}$$

> mit:

> Count = Median der gemessenen Fluoreszenzsignale
> A = untere Asymptote der Referenzkurve
> B = Steigung der Referenzkurve
> C = Wendepunkt der Referenzkurve
> D = obere Asymptote der Referenzkurve

> beschriebenen ist, wobei die Parameter A, B, C, und D experimentell mittels Titration (geometrischer Verdünnungsreihen) von Kalibratorseren ermittelbar sind, und
> (ii) ein Standardserum, welches alle zu den Sondenantigenen komplementären Antikörper in bekannten Konzentrationen enthält, und welches zur Rekalibrierung der Referenzkurve von jedem der Antikörper geeignet und vorgesehen ist.

Fig. 1

Fig. 2

Fig. 3

EP 1 630 557 A1

**Europäisches Patentamt**

**EUROPÄISCHER RECHERCHENBERICHT**

Nummer der Anmeldung

EP 05 00 8483

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | KLUTTS J S ET AL: "Evaluation of a multiplexed bead assay for assessment of Epstein-Barr Virus (EBV) immunologic status." CLINICAL CHEMISTRY, Bd. 50, Nr. 6, Suppl. S, Part 2, Juni 2004 (2004-06), Seite A54, XP002342505 & 56TH ANNUAL MEETING OF THE AMERICAN ASSOCIATION FOR CLINICAL CHEMISTRY (AACC); LOS ANGELES, CA, USA; JULY 25-29, 2004 ISSN: 0009-9147 | 1-5 | G01N33/569 G01N33/58 |
| Y | * Zusammenfassung * | 6-14 | |
| D,Y | EP 0 126 450 A (TRIPATZIS, IOANNIS, DR) 28. November 1984 (1984-11-28) * das ganze Dokument * | 12-14 | |
| Y | BIOTEST: "Biotest Infektionsdiagnostik" [Online] 9. Juni 2004 (2004-06-09), XP002343297 Gefunden im Internet: URL:http://web.archive.org/web/20040609040 048/http://www.biotest.de/de/data/pdf/biot est_diagnostik/infektionsdiagnostik/ebv/in fo-ebv-line-blot.pdf> [gefunden am 2005-09-01] * das ganze Dokument * | 6-11 | |
| Y | BAUER G: "SIMPLICITY THROUGH COMPLEXITY: INNUNOBLOT WITH RECOMBINANT ANTIGENS AS THE NEW GOLD STANDARD IN EPSTEIN-BARR VIRUS SEROLOGY" 2001, CLINICAL LABORATORY, CLIN LAB PUBLICATIONS, HEIDELBERG, DE, PAGE(S) 223-230 , XP008021514 ISSN: 1433-6510 * Seite 223 - Seite 226, Spalte 1 * | 6-8,10, 11 | |

RECHERCHIERTE SACHGEBIETE (Int.Cl.7)

G01N

-/--

Der vorliegende Recherchenbericht wurde für alle Patentansprüche erstellt

| Recherchenort | Abschlußdatum der Recherche | Prüfer |
|---|---|---|
| Den Haag | 7. September 2005 | Schlegel, B |

Europäisches
Patentamt EUROPÄISCHER RECHERCHENBERICHT

**Nummer der Anmeldung**

EP 05 00 8483

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | BAETENS D G ET AL: "Coupled particle light scattering: a new technique for serodiagnosis of Epstein-Barr virus infection." JOURNAL OF MEDICAL VIROLOGY. AUG 2001, Bd. 64, Nr. 4, August 2001 (2001-08), Seiten 519-525, XP008051656 ISSN: 0146-6615 * Seite 519 - Seite 520 * | 1-15 | |
| A | MC. PROBST ET AL.: "Bead-based multiplex analysis" LABORATORIUMSMEDIZIN 2003 GERMANY, Bd. 27, Nr. 5-6, Juni 2003 (2003-06), Seiten 182-187, XP002342506 * das ganze Dokument * | 1-15 | |
| A | B. ZIEGLER: "Infektionsserologie - Das Epstein Barr Virus"[Online] 1. September 2003 (2003-09-01), XP002342507 Gefunden im Internet: URL:http://www.fachaerzte.com/ziegler/fach informationen/ebvserologie.htm> [gefunden am 2005-08-29] * das ganze Dokument * | 1-15 | |
| P,X | KLUTTS J S ET AL: "Evaluation of a multiplexed bead assay for assessment of Epstein-Barr virus immunologic status." JOURNAL OF CLINICAL MICROBIOLOGY. NOV 2004, Bd. 42, Nr. 11, November 2004 (2004-11), Seiten 4996-5000, XP002342508 ISSN: 0095-1137 * Seite 4997, Spalte 1, Zeilen 33,34 - Spalte 2, Zeilen 9-36; Tabelle 2 * | 1-5,15 | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| Den Haag | 7. September 2005 | Schlegel, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 05 00 8483

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-09-2005

| Im Recherchenbericht angeführtes Patentdokument | Datum der Veröffentlichung | Mitglied(er) der Patentfamilie | | Datum der Veröffentlichung |
|---|---|---|---|---|
| EP 0126450 A | 28-11-1984 | DE | 3322373 A1 | 22-11-1984 |
| | | AT | 80469 T | 15-09-1992 |
| | | CA | 1248873 A1 | 17-01-1989 |
| | | DE | 3485912 D1 | 15-10-1992 |
| | | EP | 0126450 A2 | 28-11-1984 |
| | | JP | 2040626 C | 28-03-1996 |
| | | JP | 7054324 B | 07-06-1995 |
| | | JP | 60035265 A | 23-02-1985 |
| | | DK | 305084 A | 23-12-1984 |
| | | NO | 842501 A | 27-12-1984 |

EPO FORM P0461

Für nähere Einzelheiten zu diesem Anhang : siehe Amtsblatt des Europäischen Patentamts, Nr.12/82